# EUROPEAN PATENT APPLICATION

(11) **EP 1 854 457 A1**
(43) Date of publication of application: **14.11.2007**
(21) Application number: 07251956.4
(22) Date of filing: 11.05.2007
(51) Int. Cl.: A61K 9/70, A61K 8/02

(54) **Treated film strips**

(30) Priority: 12.05.2006 US 433515
(71) Applicant: Mcneil-PPC, Inc, Skillman, NJ 08558 (US)
(72) Inventor: Georgiades, Dean, East Brunswick New Jersey 08816 (US)
(74) Representative: Mercer, Christopher Paul

(57) **Abstract**

The present invention relates to treated strips or films such that the surfaces of the strips have little or no adhesion to its outer packaging. Specifically, the present invention relates to treated strips providing a strip or film layer comprising a water-insoluble particle coating on at least one surface of the strip or film layer.

## Description

### FIELD OF THE INVENTION

The present invention relates to treated strips or films such that the surfaces of the strips have little or no adhesion to its outer packaging. Specifically, the present invention relates to treated strips providing a strip or film layer comprising a water-insoluble particle coating on at least one surface of the strip or film layer.

### BACKGROUND OF THE INVENTION

Release agents or coatings are used to control or diminish the adhesion between an adhesive or tacky layer and a backing or substrate to which the adhesive or tacky layer is applied or contacts. As practical matter, using releasing agents aid in removal of adhesive substances or layers is facilitated without substantial mess or damage to the adhesive portion. Such coatings are especially relevant in the consumer products area where ease of use, convenience and product aesthetics are particularly important. Release coatings may be employed in conjunction with release films, release liners, non-stick carrier webs, and coatings for paper and polymer substrates.

In the past, silicone-based, release liquid coatings have been employed to aid release of strips or films from backing or substrate layers. However, it has been found that liquid silicones, under certain situations, are inadequate to satisfactorily prevent strip adhesion to packaging.

One advantage of the present invention is to, therefore, provide improved film or strip compositions such that when packaged, the films or strips have reduced or no adhesion to the packaging surface on which they contact.

Another advantage of the present invention is to provide packaged strips comprising a strip or film layer comprising a water-insoluble particle coating on at least one surface of the strip or film layer.

An additional advantage of the present invention is to provide packaged strips comprising a strip or film layer comprising water-insoluble film-forming agent and a water-insoluble particle coating on at least one surface of the strip or film layer.

### SUMMARY OF THE INVENTION

The present invention relates to film compositions or stand alone film strips having at least one surface, comprising;
a) at least one film forming polymer; and
b) at least one water insoluble particle coated on at least one surface of the film;
wherein at least about 15% of the surface area of the coated surface of the film is covered by the water insoluble particle.

The present invention also relates to treated film compositions comprising:
a.) a film having opposing surfaces, comprising;
   i. at least one film forming polymer; and
   ii. at least one water insoluble particle coated on at least one of the opposing surfaces of the film;
   and
b.) a package for the film
wherein the coated film surface exhibits substantially no adhesion to the package upon or after insertion therein.

The present invention also relates to treated film compositions or packaged film compositions, comprising:
a.) a film having opposing surfaces, comprising;
   i. at least one water insoluble film forming polymer; and
   ii. at least one water insoluble particle coated on at least one of the opposing surfaces of the film;
   and
b.) a package for the film
wherein the coated film surface exhibits substantially no adhesion to the package upon or after insertion therein.

Methods of making and using the above described films are also described.

Methods of using the above film compositions are also disclosed.

### DETAILED DESCRIPTION OF THE PRESENT INVENTION

The film compositions of the present invention can comprise, consist of, or consist essentially of the essential elements and limitations of the invention described herein, as well any of the additional or optional ingredients, components, or limitations described herein.

All percentages, parts and ratios are based upon the total weight of the wet film composition of the present invention, unless otherwise specified.

The term "safe and effective amount" as used herein means an amount of a compound or composition such as a topical or system active sufficient to significantly induce a positive benefit, for example, a teeth whitening, antimicrobial and/or analgesic benefit, including independently the benefits disclosed herein, but low enough to avoid serious side effects, i.e., to provide a reasonable benefit to risk ratio, within the scope of sound judgment of the skilled artisan.

The term "adhesive" as used herein, means any material or composition that is capable of sticking to the site of topical application or administration and includes, but is no limited to, mucoadhesives, pressure-sensitive adhesive (adheres upon application of pressure), moistenable adhesives (adheres in the presence of water) and tacky or sticky type adhesives (adheres upon immediate contact with a surface).

The term "substantially no adhesion" as used herein is meant to indicate that there is no pressure-sensitive tack representing the function of adhesion, supposing that the essence of tack is friction which is resistance to sliding. This pressure-sensitive tack occurs when the elastic modulus of the tacky material is 1 Mpa or less according, e.g., to Dahlquist's standard. The term can be further or alternatively understood as meaning that the adhesive exhibited by a treated or coated surface of a strip as disclosed herein is observably less than the adhesion exhibited by the surface of a strip not treated as disclosed herein.

The film compositions of the present invention, including the essential and optional components thereof, are described in detail hereinafter.

### Film-Forming Agent

The treated film or strip compositions of the present invention are film compositions formed from one or more water soluble and/or water insoluble film forming agents.

Water soluble film forming agents useful in the compositions of the present invention include, but are not limited to, pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, levan, elsinan, collagen, gelatin, zein, gluten, soy protein isolate, whey protein isolate, casein and mixtures thereof.

Water insoluble film forming agents useful in the compositions of the present invention include, but are not limited to, hydrogenated vegetable oils; natural rosins such as wood rosins and gum rosins; vegetable proteins such as corn protein, pea protein or soy protein; hydrogenated caster oil; polyvinyl chloride; shellac; polyurethane; cellulose, cellulose derivatives such as cellulose or ethylcellulose; waxes; methacrylic acid copolymers such as those sold under the Trade Mark Eudragit RS (ammoniomethacrylate copolymer), silicone or a mixture thereof.

A more detailed discussion of useful film forming agents can be found in US 2005/0196350 to Georgiades et al., published Sept. 8, 2005; US 2005/0196354 to Soshinsky, published, Sept. 8, 2005; US 2005/0196357 to Georgiades et al., published Sept. 8, 2005; and US 2005/0196358 to Georgiades et al., published Sept. 8, 2005, each of which are herein incorporated by reference.

In certain embodiments, the film forming agent is present at concentration levels from about 0.01 to about 99 wt %, preferably about 30 to about 80 wt %, optionally, from about 45 to about 70 wt % of the film and , optionally, from about 60 to about 65 wt % of the film.

### Water Insoluble Particle Releasing Agent

Once the film or strip of the present invention is formed, at least one surface of the film or strip is treated or coated with at least one water insoluble particle releasing agent. Various kinds of organic powders and inorganic powders can be used as the water-insoluble particles.

The inorganic powders or particles which are useful herein include, but are not limited to, alumina (especially microfine particles or granules), talc, magnesium stearate, titanium dioxide, barium titanate, magnesium titanate, calcium titanate, strontium titanate, zinc oxide, silica sand, clay, mica, tabular spar, diatomaceous earth, various inorganic oxide pigments, chromium oxide, cerium oxide, iron red, antimony trioxide, magnesium oxide, zirconium oxide, barium sulfate, barium carbonate, calcium carbonate, silica (colloidal or fumed), silicon carbide, silicon nitride, boron carbide, tungsten carbide, calcium hydroxyl apatite, titanium carbide, carbon black and mixtures thereof.

The organic powders or particles which are useful herein include cross- linked and non-cross-linked polymer powders, organic pigments, charge controlling agents, and waxes, for example. The cross-linked and non- cross-linked resin powders include, but are not limited to, resin powders of the styrene type, acrylic type, methacrylic type, polyethylene type, polypropylene type, silicone type, polyester type, polyurethane type, polyamide type, epoxy type, polyvinyl butyral type, rosin type, terpene type, phenol type, melamine type, and guanamine type, for example. Mixtures of any of the above organic or inorganic powders can also be used. Additional particles useful in the present invention can be found in US patents 6,475,500; US 5,611,885; and US 4,847,199 each of which are herein incorporated by reference in its entirety.

The water insoluble particles of the present invention generally have a particle size of less than 10 microns, optionally, from about 0.01 microns to about 5 microns, optionally, from about 0.1 microns to about 1 micron, and, optionally, from about 0.1 to about 0.5 microns.

In certain embodiments, the insoluble particles can include Cabosil M-5 (fumed untreated silica) supplied by Cabot, Tuscola, III.

Any dry coating method is useful in coating or applying the water insoluble product to the dried cast film. Suitable methods include, but are not limited to, electrostatic coating, dip coating, die coating, bead coating, spray coating, gravure coating, flexo printing, screen printing, offset printing or the like. While it is desirable for the water insoluble particle layer to be homogeneous, uniform and continuous, the coating may be heterogeneous, non-uniform or discontinuous. Optionally, the dry particle coating should be applied just prior packaging.

When applied to the surface of film or strip compositions of the present invention, the water insoluble particle is present at sufficient quantities to cover at least about 1% of the surface area of the strip, optionally at least about 3% of the surface area of the strip, optionally at least about 5% of the surface area of the strip, optionally at least about 10% of the surface area of the strip, optionally at least about 15% of the surface area of the strip, or optionally at least about 85% of the surface area of the strip. Optionally still, the surface coverage of the treated or coated surface can range from about 1% to about 95%, optionally from about 3% to about 85%, optionally from about 5% to about 75%.

### Film Package or Packaging

The film package is the package containing and having inner surfaces contacting the film or strip. The film package can be provided in a variety of shapes and sizes. However, it is sometimes desirable that the shape and size of the package closely conform to the shape and size of the film or strip occupying the package. The package can be provided in the form of a pouch, a box, a plastic container, an envelope, a bag, or other suitable package known in the art. A plurality of packages and film products can be bundled or otherwise provided as a set so that a sufficient supply of film products is available for multiple use. The package can be made of a material that is translucent, transparent or non-translucent. In certain embodiments, the film package has low or no moisture permeability. In other embodiments, the film package is generally impermeable to moisture. Suitable materials include, but are not limited to, plastic, paper, foil, cardboard, polymers such as polyethylene, and rubbers. A secondary package can also be provided which stores a plurality of the packages. Alternatively, the package may be made of one or more materials and may optionally have a liner. For example, a pouch could be made of foil and have a polyethylene lining.

In certain embodiments the film compositions of the present invention are dry film compositions, typically in the form of stand alone film strips. The drying may be accomplished by any conventional drying process such as, for example, air drying, heat drying, vacuum drying, freeze drying and the like. By the term "dry", as used herein, means the film compositions (typically as stand alone film strips) contain less than about 15% water, optionally less than about 10% water, optionally from about 2% to about 8% water.

### Optional Ingredients

### Disintegration Facilitator

Water insoluble particles are also useful in the compositions of the present invention as disintegration facilitators in films incorporating water insoluble polymers. Various kinds of organic powders and inorganic powders can be used as the water-insoluble particles. The above-mentioned water insoluble particles useful as releasing agents can also be incorporated separately in the film product production process as disintegration facilitators.

When incorporated in the film compositions of the present invention as a disintegration facilitator, the water insoluble particle is present at a concentration of from about 0.1% to about 25%, optionally, from about 0.5% to about 15%, and, optionally, from about 1% to about 10% by weight of the dry film composition.

### Plasticizers or Plasticizing Agents

The film compositions of the present invention optionally comprise at least one plasticizer or plasticizing agent.

Examples of suitable plasticizers include, but are not limited to, citric acid alkyl esters, glycerol esters such as glycerol monooleate and glycerol monostearate, phthalic acid alkyl esters, sebacic acid alkyl esters, sucrose esters, sorbitan esters, acetylated monoglycerides, glycerols, fatty acid esters, glycols such as propylene glycol, and polyethylene glycols 200 to 12,000 and mixtures thereof. Specific plasticizers include, but are not limited to, triethyl citrate, acetyl triethyl citrate, triacetin (glyceryl triacetate), liquid poloxamers having 50% or less polyoxyethylene (examples ranges from Pluracare L-44 from BASF to Pluracare L-121 from BASF), alkyl aryl phosphates, diethyl phthalate, tributyl citrate, dibutyl phthalate, dibutyl sebacate, polysorbate, Carbwax® series of polyethylene glycols (Union Carbide Corporation) and mixtures thereof.

In certain embodiments, the plasticizers can include mono- and di-glycerides of edible fats or oils supplied by Lonza Inc., Fair Lawn, NJ or Eastman Triacetin (food grade) supplied by Eastman Chemical Company, Kingsport, TN.

When incorporated in the film compositions of the present invention, the plasticizer is present at a concentration of from about 0.5% to about 40%, preferably from about 1% to about 20%, and most preferably from about 2% to about 10% by weight of the dry film composition.

Various topical and systemic actives can also be incorporated into the films of the present invention. The term "topical or systemic active" as used herein includes curative, prophylactic and cosmetic active substances or compositions thereof. Examples of the conditions these substances may address include, but are not limited to one or more of, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, tooth and/or gum pain, tooth sensitivity (e.g. to temperature changes), and the elimination of mouth malodour resulting from the conditions above and other causes such as microbial proliferation.. Additionally, the films of the present invention are useful for treating and/or preventing wounds, lesions, ulcers, cold sores and the like of the lips and skin generally.

Suitable topical actives for use in and around the oral cavity include any substance that is generally considered as safe for use in the oral cavity and that provides a change to the overall health of the oral cavity. The level of topical oral care active in the present invention may generally be from about 0.01 % to about 40% or, optionally, from about 0.1 % to 20% by weight of the dry film.

The topical oral care actives of the present invention may include many of the actives previously disclosed in the art. The following is a non all- inclusive list of oral care actives that may be used in the present invention.

Essential oils may be included in or associated with the films the present invention. Essential oils suitable for use herein are described in detail in US patent 6,596,298 to Leung et al., previously incorporated by reference in its entirety.

Teeth whitening actives may be included in the films of the present invention. The actives suitable for whitening are selected from the group consisting of oxalates, peroxides, metal chlorites, perforates, percarbonates, peroxyacids, and mixtures thereof. Suitable peroxide compounds include: hydrogen peroxide, calcium peroxide, sodium peroxide, carbamide peroxide, urea peroxide, sodium percarbonate and mixtures thereof. Optionally, the peroxide is hydrogen peroxide. Suitable metal chlorites include calcium chlorite, barium chlorite, magnesium chlorite, lithium chlorite, sodium chlorite and potassium chlorite. Additional whitening actives may be hypochlorite and chlorine dioxide. A preferred chlorite is sodium chlorite. The effectiveness of whitening actives can, optionally, be enhanced by means of a catalyst, i.e. a two-component peroxide- catalyst; system. Useful whitening agent catalysts or catalytic agents can be found in US 6, 440,396 to McLaughlin, Gerald, herein incorporated by reference in its entirety.

When incorporating peroxide actives, the film compositions of the present invention can, optionally, contain peroxide active stabilizers. Peroxide active stabilizers suitable for use herein include, but are not limited to polyethylene glycols such as PEG 40 or PEG 600; zinc salts such as zinc citrate; polyoxyalkylene blockpolymers (e.g., Pluronics); aminocarboxylic acids or salts thereof; glycerols; dyes such as Blue #1 or Green #3; phosphates such as phosphoric acid, sodium phosphate or sodium acid pyrophosphate; stannous salts such as stannous chloride; sodium stannate; citric acid; etidronic acid; carbomers or carboxypolymethylenes such as those of the Carbopol® seriers, butylated hydroxytoluene (BHT), ethylenediaminetetraacetic acid (EDTA) and mixtures thereof.

Anti-tartar agents useful herein include phosphates. Phosphates include pyrophosphates, polyphosphates, polyphosphonates and mixtures thereof. Pyrophosphates are among the best known for use in dental care products. Pyrophosphate ions delivered to the teeth derive from pyrophosphate salts. The pyrophosphate salts useful in the present compositions include the dialkali metal pyrophosphate salts, tetra-alkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate (K₄P₂O₇) in their unhydrated as well as hydrated forms are preferred. Anticalculus phosphates include potassium and sodium pyrophosphates; sodium tripolyphosphate; diphosphonates, such as ethane-1-hydroxy-1,1-diphosphonate; 1-azacycloheptane-1,1-diphosphonate; and linear alkyl diphosphonates; linear carboxylic acids and sodium and zinc citrate.

Agents that may be used in place of or in combination with the pyrophosphate salt include materials such as synthetic anionic polymers including polyacrylates and copolymers of maleic anhydride or acid and methyl vinyl ether (e.g. Gantrez, as described, for example, in U.S. Patent 4,627, 977, to Gaffar et al. herein incorporated by reference in its entirety, as well as e.g. polyamino propane sulfonic acid (AMPS), zinc citrate trihydrate, polyphosphates (e.g. tripolyphosphate; hexametaphosphate), diphosphonates (e.g. EHDP, AMP), polypeptides (such as polyaspartic and polyglutamic acids), and mixtures thereof.

One of more fluoride ion sources can be incorporated into the film compositions as anticaries agents. Fluoride ions are included in many oral care compositions for this purpose, and similarly may be incorporated in the invention in the same way. Detailed examples of such fluoride ion sources can be found in US patent 6,121,315 to Nair et al., herein incorporated by reference in its entirety.

Also useful herein are tooth desensitizing agents. Tooth desensitizing agents that may be used in the present invention include potassium nitrate, citric acid, citric acid salts, strontium chloride, and the like, as well as other desensitizing agents known in the art. The amount of desensitizing agent included within the dental whitening compositions of the present invention may vary according to the concentration of the potassium nitrates, the desired strength and intended treatment times. Also useful herein are remineralization technologies such as those described in US 5,981,475 to Reynolds; US 2004/0171471 to Noremberg et al.; and 2003/0219388 to Kropf et al., each of which is herein incorporated by reference in its entirety. Accordingly, if included at all, the other desensitizing agents will optionally be included in an amount in a range from about 0.1 % to about 10%, or optionally in a range of from about 1 to about 7% by weight of the dry film composition.

The compositions of the present invention may further contain a safe and effective amount of an antimicrobial agent, an anti-inflammatory agent, an anesthetic agent, and upper-respiratory active, an gastrointestinal active, a nutrient, a smoking cessation agent, an enzyme, a mouth or throat product, a histamine (H-2) antagonist and mixtures thereof.

Antimicrobial agents can also be present in the film compositions of the present invention as oral agents or topical skin and/or systemic actives. Such agents may include, but are not limited to, 5-chloro-2-(2,4-dichlorophenoxy)- phenol, commonly referred to as triclosan, chlorhexidine, alexidine, hexetidine, sanguinarine, benzalkonium chloride, salicylamide, domiphen bromide, cetylpyridium chloride (CPC), tetradecyl pyridinium chloride (TPC); N-tetradecyl-4- ethyl pyridinium chloride (TDEPC); octenidine; delmopinol, octapinol, and other piperidino derivatives, niacin preparations; zinc/stannous ion agents; antibiotics such as AUGMENTIN, amoxyicillin, tetracycline, doxycyline, minocycline, and metronidazole; and analogs, derivatives and salts of the above antimicrobial agents and mixtures thereof.

Anti-inflammatory agents can also be present in the film compositions of the present invention as oral agents or topical skin and/or systemic actives. Such agents may include, but are not limited to, non- steroidal anti-inflammatory agents or NSAIDs, such as propionic acid derivatives; acetic acid derivatives; fenamic acid derivatives; biphenylcarboxylic acid derivatives; and oxicams. All of these NSAIDS are fully described in U.S. Pat. No. 4,985,459 to Sunshine et al., issued Jan. 15, 1991, incorporated by reference herein in its entirety. Examples of useful NSAIDS include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof. Also useful are the steroidal anti-inflammatory drugs such as hydrocortisone and the like, and COX-2 inhibitors such as such as meloxicam, celecoxib, rofecoxib, valdecoxib, etoricoxib or mixtures thereof. Mixtures of any of the above anti-inflammatories may be used.

Anesthetic agent may also be incorporated herein. Examples of suitable anesthetic agents include, but are not limited to, benzocaine, betoxycaine, biphenamine, bupivacaine, butacaine, dibucaine hydrochloride, dyclonine, lidocaine, mepivacaine, procaine, propanidid, propanocaine, proparacaine, propipocaine, propofol, propoxycaine hydrochloride, pseudococaine, tetracaine hydrochloride and mixtures thereof.

Upper respiratory actives can also be used herein. Examples of such actives are sympathomimetic agents administered systemically or topically for decongestant use, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride; anti-histamines are chlorpheniramine, brompheniramine, clemastine, ketotifen, azatadine, loratadine, terfenadine, cetirizine, astemizole, tazifylline, levocabastine, diphenhydramine, temelastine, etolotifen, acrivastine, azelastine, ebastine, mequitazine, mizolastine, levocetirizine, mometasone furoate, carebastine, ramatroban, desloratadine, noberastine, selenotifen, alinastine, efletirizine, tritoqualine, norastemizole, tagorizine, epinastine, acrivastine and mixtures thereof; antitussives such as dextromethorphan, benzonatate, and guifenecin.and mixtures thereof. Other useful upper respiratory actives and be found in US patent 4,619,934, herein incorporated by reference in its entirety.

Gastro-intestinal actives can also be incorporated. Examples of suitable gastro-intestinal actives include anticholinergics, including: atropine, clidinium and dicyclomine; antacids, including aluminum hydroxide, basic bismuth salts such as bismuth subsalicylate, bismuth ranitidine citrate, bismuth subcitrate, bismuth subnitrate, aluminum or bismuth salts of polysulfated saccharides such as aluminum sucrose octasulfate or bismuth sucrose octasulfate, simethicone, calcium carbonate and magaldrate (other examples of antacids can be found in 21 CFR 331.11 which is incorporated herein by reference); H (2)-receptor antagonists, including cimetidine, famotidine, nizatidine and ranitidine; laxatives, including: bisacodyl, picosulfate, and casanthrol (other examples of laxatives can be found in the Federal Registry, Vol. 50, No. 10, Jan. 15, 1985, pp. 2152-58, which is incorporated herein by reference); gastroprotectants, including sucralfate and sucralfate humid gel; gastrokinetic and prokinetic agents including cisapride, metoclopramide and eisaprode; proton pump inhibitors including omeprazole, lanzoprazole, and antidiarrheals including: diphenoxylate and loperamide; agents which are bacteriostatic or bactericidal to the ulcer-inducing organism Heliobacter pylori such as amoxicillin, metronidazole, erythromycin, or nitrofurantoin and others agents for treating H. pylori disclosed in U.S. Pat. No. 5,256,684, which is incorporated herein by reference in its entirety; polyanionic materials useful for the treatment of ulcers and other gastrointestinal disorders including amylopectin, carragemum, sulfated dextrins, inositol hexaphosphate, or other similar agents and mixtures thereof.

Nutrients may improve the condition of the oral cavity and can be included in the oral care substances or compositions of the present invention. Examples of nutrients include minerals, vitamins, oral nutritional supplements, enteral nutritional supplements, and mixtures thereof.

Smoking cessation agents such as nicotine may also be incorporated in the film compositions of the present invention.

An individual enzyme or combination of several compatible enzymes can also be included in the oral care substance or composition of the present invention.

Enzymes are biological catalysts of chemical reactions in living devices. Enzymes combine with the substrates on which they act forming an intermediate enzyme substrate complex. This complex is then converted to a reaction product and a liberated enzyme which continues its specific enzymatic function.

Enzymes provide several benefits when used for cleansing of the oral cavity. Proteases break down salivary proteins which are absorbed onto the tooth surface and form the pellicle; the first layer of resulting plaque. Proteases along with lipases destroy bacteria by lysing proteins and lipids which form the structural component of bacterial cell walls and membranes. Dextranases break down the organic skeletal structure produced by bacteria that forms a matrix for bacterial adhesion. Proteases and amylases, not only prevent plaque formation, but also prevent the development of calculus by breaking-up the carbohydrate protein complex that binds calcium, preventing mineralisation. Enzymes useful in the present invention include any of the commercially available proteases, glucanohydrolases, endoglycosidases, amylases, nutanases, lipases and mucinases or compatible mixtures thereof. Preferred are the proteases, dextranases, endoglycosidases and multanases, most preferred being papain, endoglycidase or a mixture of dextranase and mutanase.

Other materials that can be used with the present invention include commonly known mouth and throat products. These products include, but are not limited to antifungal, antibiotic and analgesic agents.; Antioxidants are generally recognized as useful in compositions such as those of the present invention. Antioxidants that may be included in the oral care composition or substance of the present invention include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

Histamine-(H-2) receptor antagonist compounds (H-2 antagonists) may be used in the oral care composition of the present invention. As used herein, selective H-2 antagonists are compounds that block H-2 receptors, but do not have meaningful activity in blocking histamine-(H-1) receptors.

Additional useful actives can be found in US patent 6,638,528 herein incorporated by reference in its entirety.

An additional carrier material may also be added to the film composition of the present invention. These materials can be added as additional components for properties other than those previously mentioned and can include humectants and include glycerin, sorbitol, polyethylene glycol and the like. The film composition may comprise the substance itself, together with one or more substance enhancers, for example catalysts and/or potentiators to modify the release and/or activity of the substance.

The film compositions of the invention may additionally comprise additional substances such as flavours, colours, etc. which may for example be deposited onto the surface of the film or impregnated into the bulk of the film. The topical or system active is preferably teeth whitening substance. The teeth whitening substance can take the form of a peroxide-containing gel. Suitable gels may be based on glycerol containing a peroxide such as hydrogen peroxide or an organic peroxide. A suitable gel is that disclosed in US-A-3,657,413, for example that sold under the trade mark PROXIGEL by The Block Drug Company (USA) (since acquired by GlaxoSmithKline plc). Other suitable peroxide-containing gels are for example disclosed in the art references cited above. The film may have the topical or system active deposited upon its surface.

A pH adjusting agent may also be added to optimize the storage stability of the gel and to make the substance safe for the oral tissues. These pH adjusting agents, or buffers, can be any material which is suitable to adjust the pH of the oral care substance. Suitable materials include sodium bicarbonate, sodium phosphate, sodium hydroxide, ammonium hydroxide, sodium stannate, triethanolamine, citric acid, hydrochloric acid, sodium citrate, and combinations thereof. The pH adjusting agents are added in sufficient amounts so as to adjust the pH of the substance or composition to a suitable value, e.g. about 4.5 to about 11, preferably from about 5.5 to about 8.5, and more preferably from about 6 to about 7. The pH adjusting agents are generally present in an amount of from about 0.01% to about 15% and preferably from about 0.05% to about 5%, by weight of the dry film.

For example a gel may be deposited directly as a layer on a surface of a film layer as described above. Alternatively a gel may be absorbed into the above-described film layer, or impregnated into the bulk of the film material, or deposited between layers of a multiple layered film.

Methods of depositing substances upon the surfaces of film materials as described above are known, for example printing, e.g. silo screen printing, passing between impregnated rollers, dosing, a pump and nozzle, spraying, dipping etc. Methods of impregnating substances into the bulk of film materials are also known, for example admixing the substance into the strip material and then forming the strip, or exposure of the strip to the substance under conditions which cause the substance to be impregnated into the strip. Alternatively, one example of the film material may be a foam material, particularly an open-cell foam material, and the substance may be impregnated into the strip material by introducing the substance into the cells of the foam.

In one other embodiment, the film of the present invention forms the first or backing layer of a bilayer where as the second layer is a water soluble polymer film layer such as that described in US patents 6,596,298 to Leung et al. and 6,419,903 to Xu et al., both of which are herein incorporated by reference in their entirety. The bilayer film is then applied to the teeth, oral mucosa or other affected area of the skin or mouth and allowed to disintegrate over time in the presence of saliva or other aqueous media.

Additionally the film layers of the present invention can be manufactured using hot melt extrusion techniques such as that described in US patent 6,375,963 B1 to Repka et al. herein incorporated by reference in their entirety.

The device of the invention may be marked with one or more visible symbol, e.g. text matter, a trade mark, a company logo, an area of color, or an alignment feature such as a visible line or notch etc. to assist the user in applying the device to the teeth in a proper alignment. Such an alignment feature may for example comprise a symbol to show the user which way up the device should be whilst applying the device to the teeth, or which of a pair of the devices is intended for the upper teeth and which for the lower teeth. This way the device may be made more visually attractive and/or easier to use. Such symbol(s) may be applied by conventional printing or embossing processes, e.g. silk screen printing, inkjet printing etc. to the surface of the plastically deformable material opposite to the surface on which is attached the layer of an absorbent material.

If such a visible symbol is applied to this surface, a cover layer can, optionally, be applied over the symbol, for example to protect it. This cover layer may be transparent or translucent to allow visible symbols to be seen through this layer. Such a cover layer can, optionally, be applied to the film by pressing, e.g. rolling, the material of the cover layer in contact with the film.

### Methods for Delivering Topical and Systemic Actives

The present invention can be used where retention of the topical or systemic active is required for topical activity or adequate systemic absorption. The film compositions of the present invention are particularly useful for whitening tooth surfaces. Generally, the delivery of the teeth whitening actives involves topically applying the inventive film containing a safe and containing effective amount of such actives to a tooth or teeth and gums in a manner described in US patents 5,894,017; 5,891,453; 6,045,811; and 6,419,906, each of which is herein incorporated by reference in its entirety. The frequency of application and the period of use will vary widely depending upon the level of treatment required or desired, e.g., the degree of teeth whitening and/or degree of topical wound healing/disinfection desired.

When applied as a patch for the skin or mucosa, the films of the present invention can be useful for problem skin areas needing more intensive treatment or for the transdermal delivery of drugs. The patch can be occlusive, semi-occlusive or non-occlusive. The topical or systemic actives of the present invention can be contained within or coated on the surface of the film or be applied to the skin prior to application of the film. Additionally, the film can be applied wet to form a film when dried on the area of application. The film can also include actives such as chemical initiators for exothermic reactions such as those described in PCT application WO 9701313 to Burkett et al. Optionally, the film can be applied at night as a form of night therapy. Examples of useful transdermal systems are described in U.S. Patents 3,598,122; 3,598,123; 3,731,683; 3,797,494; 4,286,592; 4,314,557; 4,379,454; 4,435,180; 4,559,222; 4,568,343; 4,573,999; 4,588,580; 4,645,502; 4,704,282; 4,816,258; 4,849,226; 4,908,027; 4,943,435; and 5,004,610, all of which are herein incorporated by reference in their entirety. Actives commonly associated with transdermal delivery are disclosed in U.S. Patents 5,843,468 and 5,853,751, both of which are herein incorporated by reference in their entirety.

### Examples

The film compositions illustrated in following examples illustrate specific embodiments of the film compositions of the present invention, but are not intended to be limiting thereof. Other modifications can be undertaken by the skilled artisan without departing from the spirit and scope of this invention.

All exemplified film compositions can be prepared by conventional formulation and mixing techniques.

### Example I

The following is an example of a stand alone film of the present invention

| **Adhesive Layer** | |
|---|---|
| INGREDIENT | **AMOUNT** (weight percent) |
| DISTILLED WATER | 10.00 |
| ISO-PROPYL ALCOHOL | 79.00 |
| SILICA (fumed untreated)¹ | 4.00 |
| GLYCERIN USP SPECIAL | 2.00 |
| ZEIN² | 5.00 |

| **Coating** | |
|---|---|
| SILICA¹⁰ (fumed untreated) | |

| | |
|---|---|
| ¹ Supplied under the trade name Cabosil® by Cabot, Tuscola, I11. ² Protein from Corn, (Supplied by Freeman Industries, Tuckahoe, NY). | |

In suitable beaker, zein, silica, alcohol, glycerin and water are mixed until uniform and homogenous.

The contents of the beaker are then cast at desired thickness on a non-stick surface or sheet at room temperature to form the film or strip.

After drying, but prior to packaging, the film is passed through a roller such that at least one surface of the film or strip contacts the roller. The roller rotates through an area into which fumed silica particles are blown and dispersed. The silica particles adhere to the roller by means of electrostatic attraction. Upon contacting the surface, the silica particles are transferred from the roller to the film or strip to coat at least one of the opposing surfaces of the film or strip.

### Example II

The following is an example of a stand alone film of the present invention

| **Adhesive Layer** | |
|---|---|
| **INGREDIENT** | **AMOUNT** (weight percent) |
| ALCOHOL USP/EP | 38.00 |
| SILICA (fumed untreated)¹ | 2.00 |
| CAPOL 150² | 60.00 |

| **Coating** | |
|---|---|
| SILICA¹⁰ (fumed untreated) | |

| | |
|---|---|
| ¹ Supplied under the trade name Cabosil® by Cabot, Tuscola, III. ² Contains Ethanol, Shellac, Hydrogenated Vegetable Oil ( Coconut Origin) ( Supplied by Centerchem, Inc., Norwalk, CT). | |

In suitable beaker, Capol 150, silica, and alcohol are mixed until uniform and homogenous.

The contents of the beaker are then cast at desired thickness on a non-stick surface or sheet at room temperature to form the film or strip.

After drying, but prior to packaging, the film is passed through a roller such that at least one surface of the film or strip contacts the roller. The roller rotates through an area into which fumed silica particles are blown and dispersed. The silica particles adhere to the roller by means of electrostatic attraction. Upon contacting the surface, the silica particles are transferred from the roller to the film or strip to coat at least one of the opposing surfaces of the film or strip.

### Example III

The following is an example of a bi-layer, teeth whitening film of the present invention.

| **Adhesive Layer** | |
|---|---|
| **INGREDIENT** | **AMOUNT (weight percent)** |
| XANTHAN GUM¹ | 0.0174% w/w |
| LOCUST BEAN GUM, CLARIFIED² | 0.0348% w/w |
| CARRAGEENAN³ | 0.1740% w/w |
| PULLULAN⁴ | 4.1000% w/w |
| POVIDONE, USP K-90⁵ | 12.4000% w/w |
| SUCRALOSE⁶ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁷ | 5.7100% w/w |
| FLAVOR | 2.5890% w/w |
| POLYSORBATE 80 NF/EP⁸ | 0.3550% w/w |
| EMULSIFIER⁹ | 0.3550% w/w |
| GLYCERIN USP SPECIAL | 1.0000% w/w |

| **Backing Layer** | |
|---|---|
| PHARMACEUTICAL GLAZE, 4-LB CUT NF¹⁰ | 55.0000% w/w |
| SILICA¹¹ (fumed untreated) | 4.0000% w/w |
| ALCOHOL USP/EP | 40.0000% w/w |
| GLYCERYL STEARATE SE¹² | 1.0000% w/w |

| **Coating** | |
|---|---|
| SILICA¹⁰ (fumed untreated) | |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ PI-20 grade supplied by Hayashibara. ⁵ Polyvinylpyrrolidone, USP K-90, International Specialties Products(ISP), Wayne, NJ. ⁶ ALB CG 35% hydrogen peroxide solution, Atofina, Philadelphia, Pa. ⁷ Supplied under the trade name Splenda®, by McNeil Pharmaceuticals, NewBrunswick, NJ. ⁸ Tween 80, supplied by Quest, Hoffmann Estates, III. ⁹ Mixture of mono- and di-oleates supplied under name Atmos 300 by American Ingredients, Kansas City, Mo. ¹⁰ Shellac supplied by Mantrose Haeser Co., Attleboro, Ma. ¹¹ Supplied under the trade name Cabosil® by Cabot, Tuscola, III. ¹² Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the backing layer of the bi-layer, teeth whitening film.

The contents of beaker A is then cast at desired thickness over the above-described first layer at room temperature to form the adhesive layer of the bi-layer, teeth whitening film.

After drying, but before packaging, film is passed through a roller such the surface of the backing layer contacts the roller. The roller rotates over a pan or tray containing fumed silica particles. The silica particles adhere to the roller by means of electrostatic attraction. Upon contacting the surface, the silica particles are transferred from the roller to the backing layer to coat the surface of the backing layer.

### Example IV

The following is an example of a bi-layer, teeth whitening film of the present invention.

| **Adhesive Layer** | |
|---|---|
| *INGREDIENT* | **AMOUNT (weight percent**) |
| XANTHAN GUM¹ | 0.02308% w/w |
| LOCUST BEAN GUM, CLARIFIED² | 0.04616% w/w |
| CARRAGEENAN³ | 0.2308% w/w |
| POVIDONE, USP K-90⁴ | 16.426% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP ⁷ | 0.3550% w/w |
| EMULSIFIER⁸ | 0.3550% w/w |
| GLYCERIN USP SPECIAL | 1.0000% w/w |

| **Backing Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| **Coating** | |
|---|---|
| SILICA¹⁰ (fumed untreated) | |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the trade name Spenda®, by McNeil Pharmaceuticals, Philadelphia, Pa. ⁷ Tween 80, supplied by Quest,_Hoffmann Estates, I11. ⁸ Mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the trade name Cabosil® by Cabot, Tuscola, III. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the backing layer of the bi-layer, teeth whitening film.

The contents of beaker A is then cast at desired thickness over the above-described first layer at room temperature to form the adhesive layer of the bi-layer, teeth whitening film.

After drying, film is passed through a roller such the surface of the adhesive layer contacts the roller. The roller rotates through an area into which fumed silica particles are blown and dispersed. The silica particles adhere to the roller by means of electrostatic attraction. Upon contacting the surface, the silica particles are transferred from the roller to the adhesive layer to coat the surface of the adhesive layer. (As an additional or simultaneous step, the film can, optionally, be passed through the same roller a second time [or passed through a second roller] such the surface of the backing layer is coated in substantially the way as the adhesive layer).

### Example V

The following is an example of a bi-layer, teeth whitening film of the present invention.

| **Adhesive Layer** | |
|---|---|
| *INGREDIENT* | **AMOUNT** (**weight percent**) |
| XANTHAN GUM¹ | 0.0674% w/w |
| LOCUST BEAN GUM, CLARIFIED² | 0.0848% w/w |
| PULLULAN³ | 4.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

| **Backing Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| **Coating** | |
|---|---|
| SILICA¹⁰ (fumed untreated) | |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ PI-20 grade supplied by Hayashibara. ⁴ Polyvinylpyrrolidone, USP K-90, _ International Specialties ⁵ Products(ISP), Wayne, NJ. ⁵ ALB CG 35% hydrogen peroxide solution, Atofina, Philadelphia, Pa. ⁶ Supplied under the trade name Spenda®, by McNeil Pharmaceuticals, Philadelphia, Pa. ⁷ Tween 80, supplied by Quest, _Hoffmann Estates, III. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the trade name Cabosil® by Cabot, Tuscola, III. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the backing layer of the bi-layer, teeth whitening film.

The contents of beaker A is then cast at desired thickness over the above-described first layer at room temperature to form the adhesive layer of the bi-layer, teeth whitening film.

After drying, film is passed through a roller such the surface of the backing layer contacts the roller. The roller rotates over a pan or tray containing fumed silica particles. The silica particles adhere to the roller by means of electrostatic attraction. Upon contacting the surface, the silica particles are transferred from the roller to the backing layer to coat the surface of the backing layer.

### Example VI

The following is an example of a bi-layer, teeth whitening film of the present invention.

| **Adhesive Layer** | |
|---|---|
| *INGREDIENT* | **AMOUNT** (**weight percent**) |
| STARCH GUM¹ | 1.9674% w/w |
| GUM ARABIC² | 0.1848% w/w |
| PULLULAN³ | 2.1740% w/w |
| POVIDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

| **Backing Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| **Coating** | |
|---|---|
| SILICA¹⁰ (fumed untreated) | |

| | |
|---|---|
| ¹ Supplied under the trade name of Pure-Cote B760, supplied by Grain processing Corporation, Muscatine, IA. ² Supplied under the name Bright Gum Arabic Spray Dry FCC/NF Powder by TIC Gums, Belcamp, MD ³ PI-20 grade supplied by Hayashibara. ⁴ Polyvinylpyrrolidone, USP K-90, International Specialties Products(ISP), Wayne, NJ. ⁵ ALB CG 35% hydrogen peroxide solution, Atofina, Philadelphia, Pa. ⁶ Supplied under the trade name Splenda®, by McNeil Pharmaceuticals, Philadelphia, Pa. ⁷ Tween 80, supplied by Quest, Hoffmann Estates, III. ⁸ mixture of mono- and di-oleates supplied under name Atmos 300 by American Ingredients, Kansas City, Mo. ⁹ Shellac supplied by Mantrose Haeser Co., Attleboro, Ma. ¹⁰ Supplied under the trade name Cabosil® by Cabot, Tuscola, III. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), starch gum, gum arabic, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and Atmos 300 are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick at room temperature to form the backing layer of the bi-layer, teeth whitening film.

The contents of beaker A is then cast at desired thickness over the above-described first layer at room temperature to form the adhesive layer of the bi-layer, teeth whitening film.

After drying, film is passed through a roller such the surface of the backing layer contacts the roller. The roller rotates through an area into which fumed silica particles are blown and dispersed. The silica particles adhere to the roller by means of electrostatic attraction. Upon contacting the surface, the silica particles are transferred from the roller to the backing layer to coat the surface of the backing layer.

## Claims

1. A treated film composition comprising:
a.) a film having opposing surfaces, comprising;
i. at least one film forming polymer; and
ii. at least one water insoluble particle coated on at least one opposing surface of the film;
and
b.) a package for the film
wherein the coated film surface exhibits substantially no adhesion to the package upon insertion therein.

2. The treated film according to Claim 1, wherein the film forming polymer is selected from the group consisting of water soluble polymers, water insoluble polymers and mixtures thereof.

3. The treated film according to Claim 2, wherein the water soluble polymer is selected from the group consisting of pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, levan, elsinan, collagen, gelatin, zein, gluten, soy protein isolate, whey protein isolate, casein and mixtures thereof.

4. The treated film according to Claim 3, wherein the water soluble polymer is a mixture of water soluble polymers.

5. The treated film according to Claim 2, wherein the water insoluble polymer is selected from the group consisting of hydrogenated vegetable oils; natural rosins such as wood rosins and gum rosins; vegetable proteins such as corn protein, pea protein or soy protein; hydrogenated caster oil; polyvinyl chloride; shellac; polyurethane; cellulose, cellulose derivatives; waxes; methacrylic acid copolymers, silicone or a mixture thereof.

6. The treated film according to Claim 5, wherein the water insoluble particle is selected from the group consisting of inorganic particles, organic particles or mixtures thereof.

7. The treated film according to Claim 6, wherein the water insoluble particle is selected is an inorganic particle selected from the group consisting of alumina, talc, magnesium stearate, titanium dioxide, barium titanate, magnesium titanate, calcium titanate, strontium titanate, zinc oxide, silica sand, clay, mica, tabular spar, diatomaceous earth, various inorganic oxide pigments, chromium oxide, cerium oxide, iron red, antimony trioxide, magnesium oxide, zirconium oxide, barium sulfate, barium carbonate, calcium carbonate, silica (colloidal or fumed), silicon carbide, silicon nitride, boron carbide, tungsten carbide, calcium hydroxyl apatite, titanium carbide, carbon black and mixtures thereof.

8. The treated film according to Claim 7, wherein the water insoluble particle is silica.

9. The treated film according to Claim 6, wherein the water insoluble particle is selected is an organic particle selected from the group consisting of cross- linked and non-cross-linked polymer powders, organic pigments, charge controlling agents, waxes and mixtures thereof.

10. A treated film composition comprising:
a.) a film having opposing surfaces, comprising;
i. at least one water insoluble film forming polymer; and
ii. at least one water insoluble particle coated on at least one opposing surface of the film;
and
b.) a package for the film
wherein the coated film surface exhibits substantially no adhesion to the package upon insertion therein.

11. A treated film composition comprising:
a.) a film having opposing surfaces, comprising;
i. at least one water soluble film forming polymer; and
ii. at least one water insoluble particle coated on at least one opposing surface of the film;
and
b.) a package for the film
wherein the coated film surface exhibits substantially no adhesion to the package upon insertion therein.

12. A film composition having at least one surface having a surface area, comprising;
a) at least one film forming polymer; and
b) at least one water insoluble particle coated on at least one surface of the film;
wherein at least about 15% of the surface area of the coated surface of the film is covered by the water insoluble particle.

13. A method of preventing or reducing the adhesion of a stand alone films or strips to packaging, comprising the steps of:
a.) providing a film or strip having opposing surfaces; and
b.) treating the film or strip by coating at least one of the opposing surfaces with a water insoluble particle.

14. The method of Claim 13, wherein the treated film or strip is placed in contact with the inner surfaces of a packaging container for the film or strip.
